(19) Europäisches Patentamt
European Patent Office
Office européen des brevets

(11) EP 3 584 578 B1

(12) **EUROPEAN PATENT SPECIFICATION**

(45) Date of publication and mention
of the grant of the patent:
**12.05.2021 Bulletin 2021/19**

(21) Application number: **18754102.4**

(22) Date of filing: **26.01.2018**

(51) Int Cl.:
*G01N 33/558* (2006.01)     *G01N 35/00* (2006.01)

(86) International application number:
**PCT/JP2018/002420**

(87) International publication number:
**WO 2018/150846 (23.08.2018 Gazette 2018/34)**

(54) **IMMUNOLOGICAL TEST DEVICE AND OPERATION METHOD FOR SAME**

IMMUNOLOGISCHE TESTVORRICHTUNG UND BETRIEBSVERFAHREN DAFÜR

DISPOSITIF DE TEST IMMUNOLOGIQUE ET SON PROCÉDÉ DE FONCTIONNEMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.02.2017 JP 2017026869**

(43) Date of publication of application:
**25.12.2019 Bulletin 2019/52**

(73) Proprietor: **FUJIFILM Corporation
Tokyo 106-8620 (JP)**

(72) Inventors:
• **YUMBE, Hirona
Ashigarakami-gun
Kanagawa 258-8538 (JP)**
• **SATO, Keiichiro
Ashigarakami-gun
Kanagawa 258-8538 (JP)**

(74) Representative: **Klunker IP
Patentanwälte PartG mbB
Destouchesstraße 68
80796 München (DE)**

(56) References cited:
WO-A1-2010/058472    WO-A1-2016/006922
JP-A- 2011 174 865    JP-A- 2012 103 150
JP-A- 2013 213 803    US-B2- 8 474 303

• M. Mori ET AL: "Development of a highly sensitive
immunochromatographic detection kit for
seasonal influenza virus using silver
amplification", Fuji Research & Development, 27
February 2012 (2012-02-27), pages 1-11,
XP055198514, Ushijima Retrieved from the
Internet:
URL:http://www.fujifilm.com/about/research
/report/057/pdf/index/ff_rd057_002_en.pdf
[retrieved on 2015-06-25]
• Fujifilm: "FUJI DRI-CHEM IMMUNO AG FluAB", ,
1 July 2016 (2016-07-01), XP055658115, Retrieved
from the Internet:
URL:https://www.fujifilm.eu/fileadmin/coun
tries/europe/products/Medical/Brochures/FD
C/FDC_Human/Immuno_AG1_Cartridges_IfU_Fl
uA B.pdf [retrieved on 2020-01-15]

**Description**

**BACKGROUND OF THE INVENTION**

1. Field of the Invention

The present invention relates to an immunological test apparatus.

2. Description of the Related Art

In recent years, in the medical field, an immunological test apparatus is widely used. The immunological test apparatus can easily and quickly perform a qualitative reaction test for testing positive and negative of an infectious disease, such as influenza, and the like. In the immunological test apparatus, a carrier holding a reagent that is combined with a test substance to be colored is used. In the case of a qualitative reaction test for an infectious disease, the test substance is an antigen and the reagent is an antibody. The carrier is, for example, a nitrocellulose film, and is usually provided to a user, such as a medical staff member, in the form of a cartridge housed in a dedicated case. A sample for determining whether or not a test substance is present, for example, a sample in which a body fluid such as pharyngeal swab or nasal swab is mixed with a predetermined solution, is dropped onto the carrier.

The immunological test apparatus receives a cartridge having a sample dropped onto a carrier, images a portion of the reagent with an imaging element, and measures the coloration state (density, chromaticity) of the reagent based on an imaging signal obtained by the imaging. Then, it is determined whether or not the test substance is present in the sample based on the measurement result, and the determination result is displayed for a user.

In a case where only a very small amount of test substance is present in the sample, the density of coloration of the reagent is naturally very low. In the immunological test apparatus, even in a case where the density of coloration of such a reagent is very low, it is required to correctly determine that the test substance is present in the sample. Therefore, in order to meet this demand, for example, an immunological test apparatus disclosed in JP2012-103150A has been proposed.

The immunological test apparatus disclosed in JP2012-103150A performs processing (hereinafter, sensitization processing) for spreading a chemical solution for sensitizing the state of coloration (increasing the density of coloration) of a reagent, for example, a solution of a silver ion-containing compound, such as silver nitrate, onto a carrier. The chemical solution is stored in a pot provided in a cartridge. A chemical solution spreading unit that spreads a chemical solution onto a carrier by crushing a pot is provided in the immunological test apparatus.

US 8,474,303 B2 discloses a chromatographic measurement apparatus for measuring a color development state of an insoluble carrier with a sample solution and a label solution developed thereon to test a test article, the insoluble carrier including a test detection area where a material that binds specifically to a test article is immobilized and a control detection area used for determining an end of measurement. The chromatographic measurement apparatus includes: a determining unit for carrying out determination of validity and determination of necessity of amplification of a test result of the test article; and an amplifying unit for amplifying the color development state according to the result of the determination of necessity of amplification by the determining unit. The amplification is carried out only when it is determined that amplification is necessary.

M. Mori ET AL, "Development of a highly sensitive immunochromatographic detection kit for seasonal influenza virus using silver amplification" discloses a method for increasing sensitivity of immunochromatography by silver amplification.

Fujifilm, "FUJI DRI-CHEM IMMUNO AG FluAB" discloses a method and kit for detection of influenza A and B virus antigens in nasopharyngeal swab.

**SUMMARY OF THE INVENTION**

In general, a stop button for giving an instruction to stop the test is provided in the immunological test apparatus. By operating the stop button, the user can stop the test at any timing and take out the cartridge from the immunological test apparatus.

Here, the chemical solution for sensitization processing spread on the carrier is absorbed by the carrier with the passage of time. During this time, the chemical solution for sensitization processing spread on the carrier remains as droplets on the carrier. In a case where the stop button is operated in a state in which the droplets remain on the carrier and the cartridge is taken out from the immunological test apparatus, there is a possibility that remaining droplets will adhere to the user's hand or the like.

It is an object of the present invention to provide an immunological test apparatus and an operation method thereof capable of preventing a chemical solution for sensitization processing from adhering to a user.

In order to solve the aforementioned problems, an immunological test apparatus of the present invention is an immunological test apparatus that receives a carrier holding a reagent, which is combined with a test substance to be colored, and having a sample dropped thereon, measures a state of the coloration, and determines whether or not the test substance is present in the sample based on a result of the measurement. The immunological test apparatus comprises: a chemical solution spreading unit that performs sensitization processing for spreading a chemical solution for sensitizing a state of the coloration onto the carrier; by increasing the density of coloration; wherein the chemical solution includes a reducing solution and a sensitizing solution; a driving controller that controls driving of the chemical solution spreading unit; a stop button that gives an instruction to stop a test; and a function switching unit that performs switching between activation and deactivation of a function of the stop button and that deactivates the function of the stop button in a case where the driving controller drives the chemical solution spreading unit to start the sensitization processing.

It is preferable that the function switching unit is a display controller that performs control to display the stop button on a touch panel and that the stop button is not displayed in a case where the sensitization processing is started.

It is preferable that the driving controller drives the chemical solution spreading unit to start the sensitization processing in a case where a determination result indicating that the test substance is present in the sample is not obtained during a period from test start to a set time set in advance.

It is preferable that there are two types of the chemical solution that are a solution of a divalent iron ion-containing compound and a solution of a silver ion-containing compound. It is preferable that the reagent contains an antibody of influenza virus.

An operation method of an immunological test apparatus of the present disclosure is an operation method of an immunological test apparatus that receives a carrier holding a reagent, which is combined with a test substance to be colored, and having a sample dropped thereon, measures a state of the coloration, and determines whether or not the test substance is present in the sample based on a result of the measurement. The operation method comprises: a sensitization processing step of spreading a chemical solution for sensitizing a state of the coloration onto the carrier; and a function deactivation step of deactivating a function of a stop button for giving an instruction to stop a test in a case where the sensitization processing step is started.

According to the present invention, in a case where the sensitization processing for spreading the chemical solution for sensitizing the state of the coloration onto the carrier is started, the function of the stop button for giving an instruction to stop the test is deactivated. Therefore, it is possible to provide an immunological test apparatus and an operation method thereof capable of preventing the chemical solution for sensitization processing from adhering to the user.

## BRIEF DESCRIPTION OF THE DRAWINGS

Fig. 1 is a perspective view showing the external appearance of an immunological test apparatus.
Fig. 2 is a partially broken side view of the immunological test apparatus.
Fig. 3 is a diagram showing a chemical solution spreading unit and the inside of a cartridge.
Fig. 4 is a diagram showing how a sample moves from a dropping port to a test region.
Fig. 5 is a diagram showing how a reducing solution is spread to the test region.
Fig. 6 is a diagram showing how a sensitizing solution is spread to the test region.
Fig. 7 is a table showing a test substance and a reagent of each test line.
Fig. 8 is a table showing a reducing solution and a sensitizing solution.
Fig. 9 is a block diagram of the immunological test apparatus.
Fig. 10 is a diagram showing a display screen displayed on a touch panel.
Fig. 11 is a diagram showing a display state of a circular display region in a case where a cartridge is loaded.
Fig. 12 is a diagram showing a display state of a circular display region after a test is started.
Fig. 13 is a diagram showing a display state of a circular display region in a case where a first determination result indicating that a test substance DA is present in a sample is obtained.
Fig. 14 is a diagram showing a display state of a circular display region in a case where a small circle is operated in the display state shown in Fig. 13.
Fig. 15 is a diagram showing a display state of a circular display region in a case where a second determination result indicating that a test substance DB is present in a sample is obtained.
Fig. 16 is a diagram showing a display state of a circular display region in a case where a small circle is operated in the display state shown in Fig. 15.
Fig. 17 is a diagram showing a display state of a circular display region in a case where both the first determination result indicating that the test substance DA is present in a sample and the second determination result indicating that the test substance DB is present in a sample are obtained.
Fig. 18 is a diagram showing a display state of a circular display region in a case where sensitization processing is started from the display state shown in Fig. 12.
Fig. 19 is a diagram showing a display state of a circular display region in a case where sensitization processing is started from the display state shown in Fig. 13.
Fig. 20 is a diagram showing a display state of a circular display region in a case where sensitization processing is started from the display state shown in Fig. 15.
Fig. 21 is a diagram showing a timing at which switching between display (function activation) and non-display (function deactivation) of a stop button occurs.
Fig. 22 is a flowchart showing the procedure of processing of the immunological test apparatus.

Fig. 23 is a flowchart showing the procedure of processing of the immunological test apparatus.

Fig. 24 is a block diagram of an immunological test apparatus of an an aspect according to the second disclosure.

Fig. 25 is a table showing a test substance and a reagent of each test line in the aspect according to the second disclosure.

Fig. 26 is a diagram showing a display state of a circular display region after a test is started in the an aspect according to the second disclosure.

Fig. 27 is a flowchart showing the procedure of processing of the immunological test apparatus of the an aspect according to the second disclosure.

## DESCRIPTION OF THE PREFERRED EMBODIMENTS

[Embodiment according to the first invention]

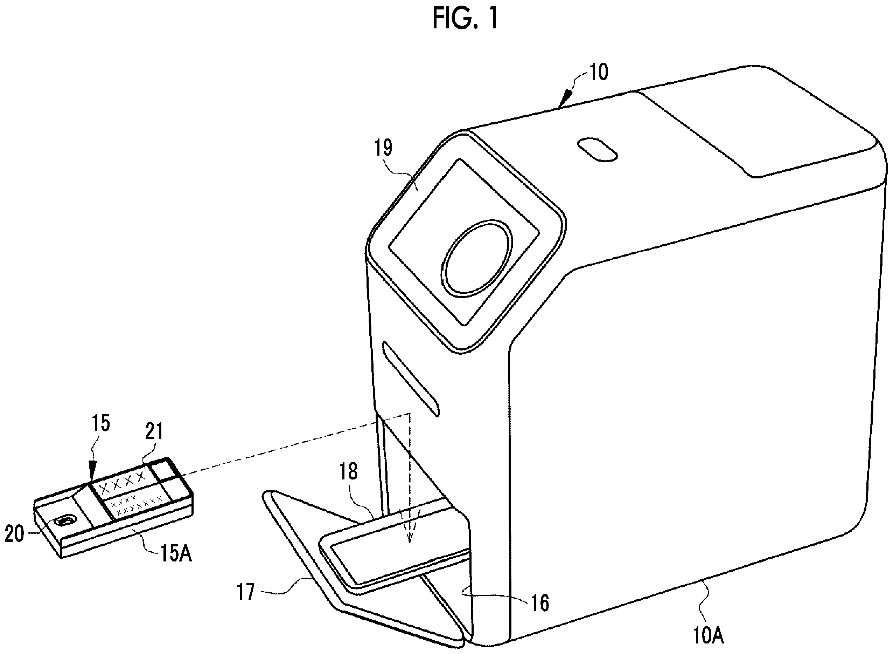

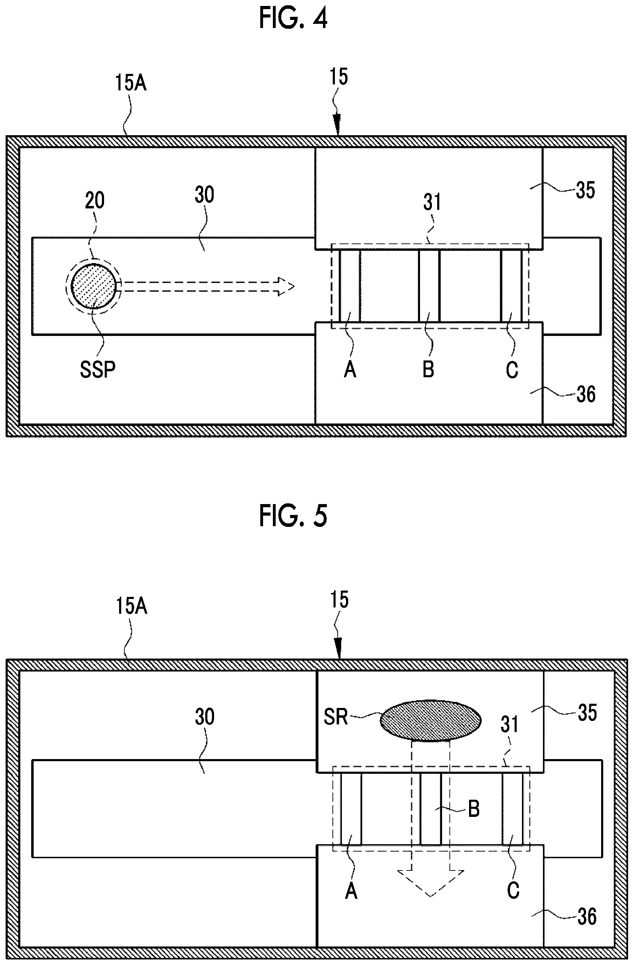

In Fig. 1, an immunological test apparatus 10 is provided in a medical institution, such as a hospital, for example. The immunological test apparatus 10 has an apparatus main body 10A. The immunological test apparatus 10 receives a dedicated cartridge 15, on which a sample SSP (refer to Fig. 4) is dropped, in the apparatus main body 10A and performs an immunological test on the sample SSP. The sample SSP is, for example, a solution obtained by mixing body fluid, such as pharyngeal swab or nasal swab collected from a patient, with a predetermined extract.

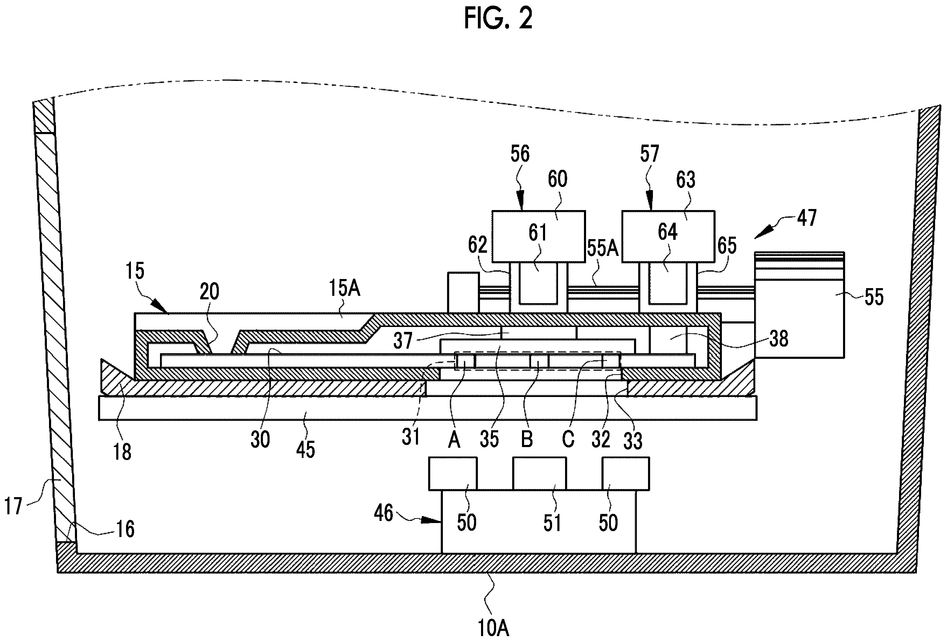

A rectangular opening 16 for receiving the cartridge 15, an openable lid 17 for covering the opening 16, and a tray-shaped cartridge loading unit 18 onto which the cartridge 15 is loaded are provided in the front lower portion of the apparatus main body 10A. The cartridge loading unit 18 slides in the opening and closing direction of the lid 17 in conjunction with the opening and closing of the lid 17. More specifically, the cartridge loading unit 18 slides between the exposed position shown in Fig. 1, which is mostly exposed from the opening 16 in a case where the lid 17 is opened, and the housing position shown in Fig. 2, which is housed in the apparatus main body 10A in a case where the lid 17 is closed.

The front upper portion of the apparatus main body 10A is an inclined surface portion, and a touch panel 19 is attached to the inclined surface portion. An operation instruction from the user, such as a medical staff member, is input to the touch panel 19, and information regarding the immunological test is displayed. Examples of the operation instruction include a test start instruction, a test stop instruction, and an instruction to print out a determination result indicating that a test substance is present in the sample SSP. The information regarding the immunological test includes patient identification (ID) for identifying a patient from whom the sample SSP is collected, an elapsed time from the start of the test, a determination result, and the like.

The cartridge 15 has a case 15A in which a carrier 30 (refer to Fig. 2) is housed. On the upper surface of the case 15A, a reverse conical dropping port 20 through which the sample SSP is dropped is provided. In addition, a label 21 on which a patient ID and the like are written is attached to the upper surface of the case 15A.

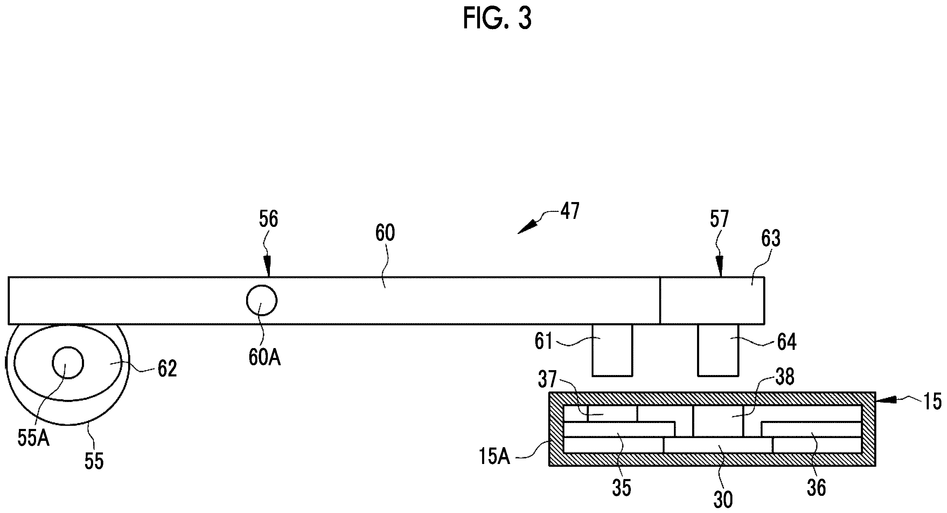

As shown in the cutaway view of the cartridge 15 of Figs. 2 and 3, a belt-shaped carrier 30 is housed in the case 15A along the longitudinal direction. The carrier 30 is, for example, a nitrocellulose film, and has a test region 31 formed by two test lines A and B and a control line C. On the test line A, a reagent RA that is combined with a test substance DA to be colored is fixed. On the test line B, a reagent RB that is combined with a test substance DB to be colored is fixed. The control line C is a line for determining whether or not the amount of sample SSP appropriate for measurement has normally flowed through the carrier 30, and makes a color in a case where the amount of sample SSP appropriate for measurement has normally flowed through the carrier 30.

On the lower surface of the case 15A, an observation window 32 for observing the coloration state of the test lines A and B and the control line C is formed. A similar observation window 33 is also formed in the cartridge loading unit 18 at a position corresponding to the observation window 32 of the case 15A.

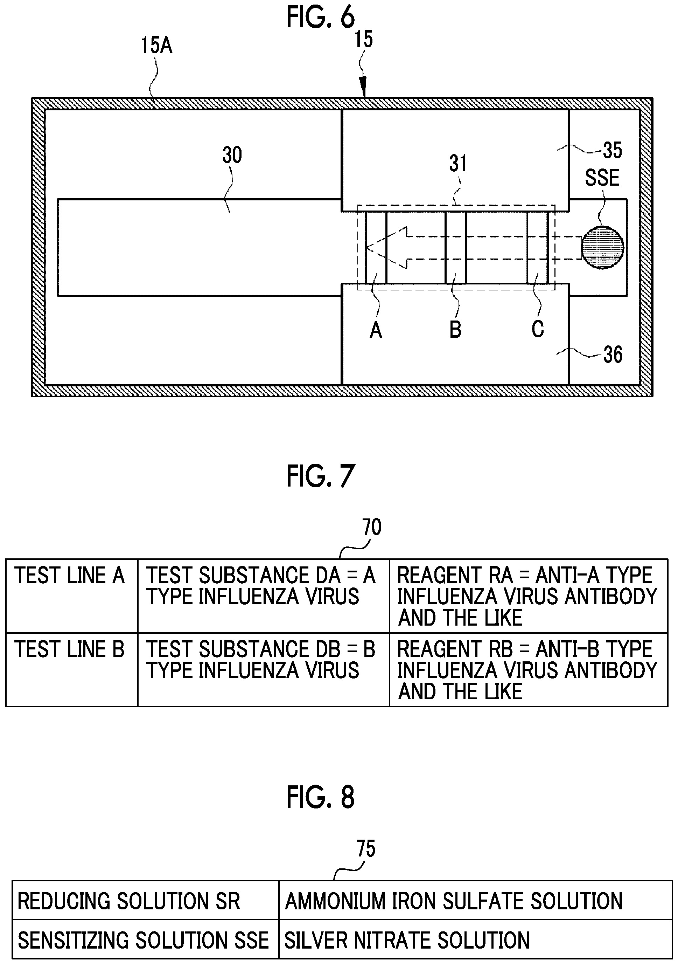

In addition to the carrier 30, a solution feeding pad 35, a solution absorbing pad 36 (not shown in Fig. 2, refer to Fig. 3), a reducing solution pot 37, and a sensitizing solution pot 38 are housed in the case 15A. The solution feeding pad 35 and the solution absorbing pad 36 are disposed at positions interposing the carrier 30 from both sides in the test region 31 (refer to Fig. 4 and the like). The reducing solution pot 37 is disposed above the solution feeding pad 35, and stores a reducing solution SR (refer to Fig. 5) corresponding to a chemical solution for sensitizing the coloration states of the reagents RA and RB. The sensitizing solution pot 38 is disposed above the end portion of the carrier 30 on the label 21 side, and stores a sensitizing solution SSE (refer to Fig. 6) corresponding to a chemical solution.

In Fig. 2, a guide rail 45, a measurement unit 46, and a chemical solution spreading unit 47 are provided in the apparatus main body 10A. The guide rail 45 guides a slide between the exposed position and the housing position of the cartridge loading unit 18.

In a case where the cartridge 15 is located at the housing position, the measurement unit 46 is disposed at a position facing the observation window 32 of the case 15A and the observation window 33 of the cartridge loading unit 18. The measurement unit 46 includes a pair of light sources 50 for emitting light to the test region 31, which is formed by the test lines A and B and the control line C, through the observation windows 32 and 33 and an imaging element 51 for imaging the test region 31.

The light source 50 is, for example, a module in which a light emitting diode (LED) is built, and emits white light. The light source 50 may emit monochromatic light as long as the chromaticity before and after sensitization processing, which will be described later, can be distinguished. The light source 50 can also be configured by a plurality of modules that emit monochromatic light components having different wavelengths. The imaging element 51 is, for example, a line sensor in which a plurality of photodiodes are linearly arranged or an area sensor in which a plurality of photodiodes are arranged in a matrix, and outputs an imaging signal corresponding to the amount of light received by the photodiodes.

The chemical solution spreading unit 47 performs sensitization processing for spreading the reducing solution SR and the sensitizing solution SSE, which are chemical solutions for sensitizing the coloration states of the reagents RA and RB, on the carrier 30. The chemical solution spreading unit 47 has a motor 55, a first pressing unit 56, and a second pressing unit 57. The motor 55 is shared by the pressing units 56 and 57.

In Fig. 3, the first pressing unit 56 has a first arm 60 rotatable around a shaft 60A like a seesaw, a first pressing piece 61 fixed to a lower portion of the distal end of the first arm 60, and a first cam 62 disposed on the lower side of the rear end of the first arm 60. The first cam 62 is connected to a driving shaft 55A rotated by the motor 55 so as to be able to be connected and disconnected through an electromagnetic clutch or the like (not shown), for example. In a case where the first cam 62 rotates, the rear end of the first arm 60 is pushed up, and the first pressing piece 61 at the distal end is lowered. Similarly, the second pressing unit 57 has a second arm 63, a second pressing piece 64, and a second cam 65 (refer to Fig. 2), and the second pressing piece 64 is lowered by the rotation of the second cam 65.

The first pressing piece 61 is disposed immediately above the reducing solution pot 37 in a case where the cartridge 15 is located at the housing position. In a case where the first pressing piece 61 is lowered, the reducing solution pot 37 is crushed from the outside of the case 15A by the first pressing piece 61, and the reducing solution SR is spread from the reducing solution pot 37. On the other hand, the second pressing piece 64 is disposed immediately above the sensitizing solution pot 38 in a case where the cartridge 15 is located at the housing position. In a case where the second pressing piece 64 is lowered, the sensitizing solution pot 38 is crushed from the outside of the case 15A by the second pressing piece 64, and the sensitizing solution SSE is spread from the sensitizing solution pot 38.

Although not shown, an information reading unit for reading the information written on the label 21, a printer for printing out the determination result on a predetermined sheet, and the like are provided in the apparatus main body 10A. Similarly to the measurement unit 46, the information reading unit is configured to include a light source that emits light to the label 21 and an imaging element that images the label 21. The information reading unit is disposed at a position facing the label 21 in a case where the cartridge 15 is located at the housing position.

In Fig. 4, in the immunological test, first, the sample SSP is dropped onto the carrier 30 through the dropping port 20. The sample SSP moves from the dropping port 20 onto the carrier 30 toward the test region 31. The sample SSP reaches the test line A, then reaches the test line B, and finally reaches the control line C. Here, in a case where the test substance DA is present in the sample SSP, the reagent RA of the test line A is colored. In a case where the test substance DB is present in the sample SSP, the reagent RB of the test line B is colored.

The density of coloration of the reagents RA and RB is correlated with the amount of test substances DA and DB present in the sample SSP. That is, the density of coloration of the reagents RA and RB is low in a case where only a small amount of test substances DA and DB is present in the sample SSP, and the density of coloration of the reagents RA and RB is high in a case where a large amount of test substances DA and DB is present in the sample SSP.

A region where a labeled substance is present is provided between the dropping port 20 of the carrier 30 and the test region 31. In the sample SSP, the labeled substance is mixed during movement from the dropping port 20 to the test region 31. The labeled substance is captured by the control line C, and accordingly the control line C is colored. Therefore, in a case where the sample SSP reaches the control line C after a predetermined time TE (for example, 15 minutes) has passed from dropping of the sample SSP, the control line C is colored regardless of the presence or absence of coloration of the reagents RA and RB of the test lines A and B. In a case where the coloration of the control line C cannot be checked even after the time TE has passed, an error is determined.

In Fig. 5, in a case where the first pressing unit 56 is driven and the reducing solution pot 37 is crushed by the first pressing piece 61, the reducing solution SR is dropped from the reducing solution pot 37 onto the solution feeding pad 35. The timing at which the reducing solution SR is dropped onto the solution feeding pad 35 is the timing of the start of the sensitization processing. The reducing solution SR is sent to the test region 31 along the short direction of the case 15A and absorbed by the solution absorbing pad 36.

In Fig. 6, in a case where the second pressing unit 57 is driven and the sensitizing solution pot 38 is crushed by the second pressing piece 64, the sensitizing solution SSE is dropped from the sensitizing solution pot 38 onto the end portion of the carrier 30 on the label 21 side. The sensitizing solution SSE is sent to the test region 31 along the longitudinal direction of the case 15A.

As shown in a table 70 of Fig. 7, in this example, the test substance DA is A type influenza virus, and the test substance DB is B type influenza virus. The reagent RA is anti-A type influenza virus antibody, anti-A type influenza virus antibody bonding gold colloid, anti-A type influenza virus antibody bonding colored latex, or the like. The reagent RB is anti-B type influenza virus antibody, anti-B type influenza virus antibody bonding gold colloid, anti-B type influenza virus antibody bonding colored latex, or the like. Thus, the reagents RA and RB contain antibodies of influenza virus.

In addition, as shown in a table 75 of Fig. 8, in this example, the reducing solution SR is a solution of ammonium iron sulfate that is a divalent iron ion-containing compound. On the other hand, the sensitizing solution SSE is a solution of silver nitrate that is a silver ion-containing compound. As described above, there are two types of chemical solutions of a solution of a divalent iron ion-containing compound (reducing solution SR) and a solution of a silver ion-containing compound (sensitizing solution SSE). The silver ion-containing compound may be silver acetate, silver lactate, silver butyrate, silver thiosulfate, or the like.

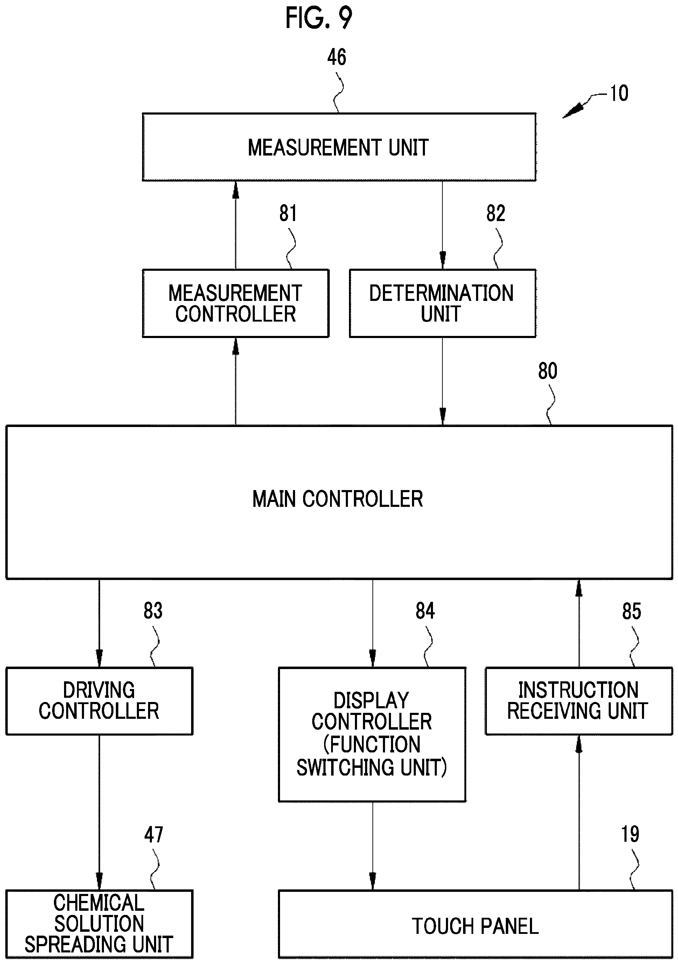

In Fig. 9, a main controller 80 performs overall control of the immunological test apparatus 10. A measurement controller 81, a determination unit 82, a driving controller 83, a display controller 84, and an instruction receiving unit 85 are connected to the main controller 80.

The measurement controller 81 controls the driving of the measurement unit 46. More specifically, the measurement controller 81 drives the light source 50 and the imaging element 51 of the measurement unit 46 at predetermined time intervals (for example, one minute intervals), so that the light source 50 emits light to the test region 31 and the imaging element 51 images the test region 31.

The determination unit 82 receives an imaging signal from the imaging element 51 of the measurement unit 46 at predetermined intervals. The determination unit 82 determines whether or not the test substances DA and DB are present in the sample SSP from the coloration state (density, chromaticity) of the reagents RA and RB derived based on the imaging signal. For example, in a case where the density and chromaticity of the coloration of the reagents RA and RB exceed threshold values set in advance, the determination unit 82 determines that the test substances DA and DB are present in the sample SSP.

In a case where it is determined that the test substance DA is present in the sample SSP, the determination unit 82 outputs a first determination result indicating that the test substance DA is present in the sample SSP to the main controller 80. In a case where it is determined that the test substance DB is present in the sample SSP, the determination unit 82 outputs a second determination result indicating that the test substance DB is present in the sample SSP to the main controller 80. In addition, the determination unit 82 determines whether or not the measurement has ended correctly from the coloration state of the control line C, and outputs the determination result to the main controller 80.

The test substance DA is A type influenza virus, and the test substance DB is B type influenza virus. Therefore, the first determination result indicates that the patient, from whom the sample SSP has been collected, is infected with A type influenza virus (A type influenza virus positive). The second determination result indicates that the patient, from whom the sample SSP has been collected, is infected with B type influenza virus (B type influenza virus positive).

Assuming that the intensity of light emitted from the light source 50 to the test region 31 is I and the intensity of reflected light from the test region 31 imaged by the imaging element 51 is IR, the density OC of the coloration of the reagents RA and RB is defined by the following Equation (1).

$$OC = \log_{10}(IR/I) \cdots (1)$$

The chromaticity is a quantitative expression of the hue and the saturation of the color of the reagents RA and RB, and is calculated from the imaging signal using a known calculation equation. As a color system of chromaticity, a general commission internationale de l'eclairage (CIE) color system can be used.

The driving controller 83 controls the driving of the chemical solution spreading unit 47. In practice, the driving controller 83 is a driver of the motor 55 of the chemical solution spreading unit 47. The driving controller 83 drives the chemical solution spreading unit 47 to start sensitization processing.

The main controller 80 counts an elapsed time TP from the start of the test. The main controller 80 outputs a driving command to the driving controller 83 in a case where at least one of the first determination result or the second determination result is not received from the determination unit 82 even though the elapsed time TP exceeds a set time TS (for example, 12 minutes) set in advance (TP > TS). The driving controller 83 drives the chemical solution spreading unit 47 in response to the driving command. That is, in a case where both the first determination result and the second determination result or either the first determination result or the second determination result is not obtained during a period from the start of the test to the set time TS (0 < TP ≤ TS), the sensitization processing is started. Conversely, in a case where both the first determination result and the second determination result are obtained during the period from the start of the test to the set time TS, the sensitization processing is not performed.

In a case where the elapsed time TP becomes the time TE taken for the sample SSP to reach the control line C (TP = TE) and a determination result indicating that the measurement has ended correctly is received from the determination unit 82, the main controller 80 ends the test. In a case where neither the first determination result nor the second determination result is obtained at the end of the test, it is understood that the patient from whom the sample SSP has been collected is not infected with both A type influenza virus and B type influenza virus (A type influenza virus negative and B type influenza virus negative). In a case where the test has ended, the user can take out the cartridge 15 from the immunological test apparatus 10.

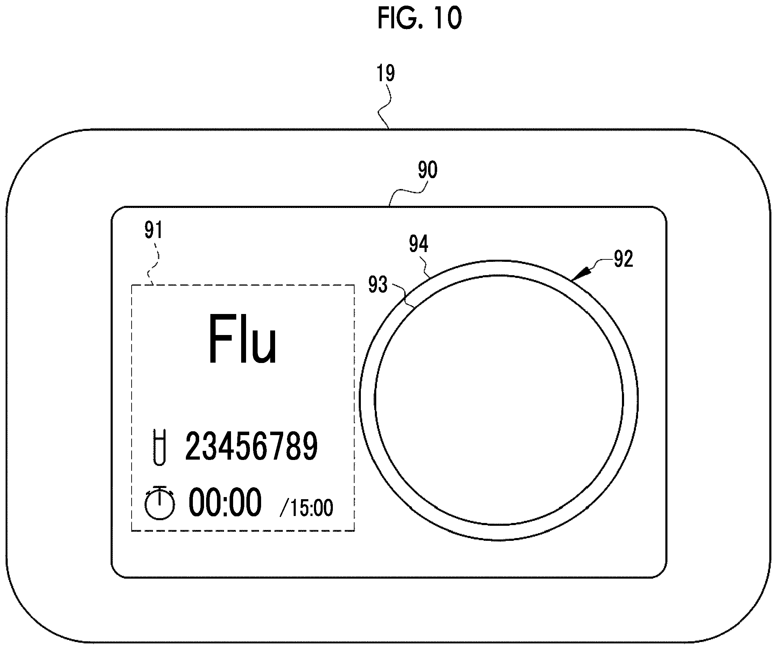

The display controller 84 controls the display of a display screen 90 (refer to Fig. 10) on the touch panel 19. The instruction receiving unit 85 receives an operation instruction input by the user through the touch panel 19.

As shown in Fig. 10, the display screen 90 displayed on the touch panel 19 has a character display region 91 for displaying a patient ID or the elapsed time TP and a circular display region 92. The circular display region 92 is configured to include two circles of a small circle 93 and a large circle 94 whose centers are the same.

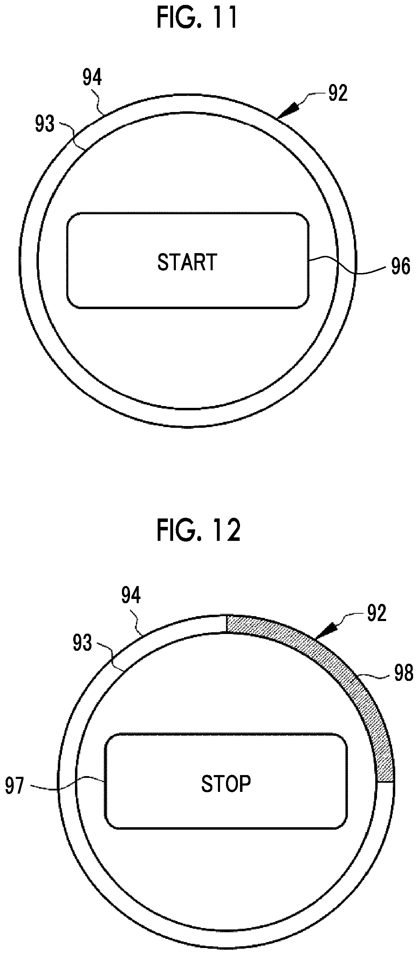

In a case where the cartridge 15 having the sample SSP dropped thereon is loaded into the cartridge loading unit 18 and the lid 17 is closed, the display controller 84 displays a "START" mark 96 at the center of the small circle 93 so that the small circle 93 functions as a start button for giving an instruction to start the test, as shown in Fig. 11. In a case where the small circle 93 is operated with the user's finger in this display state, the instruction receiving unit 85 receives a test start instruction. In a case where the test start instruction is received by the instruction receiving unit 85, the main controller 80 starts counting the elapsed time TP, and the measurement controller 81 causes the measurement unit 46 to start measurement.

After the test start instruction is received by the instruction receiving unit 85 and the test is started, the display controller 84 displays a "STOP" mark 97 at the center of the small circle 93 so that the small circle 93 functions as a stop button for giving an instruction to stop the test, as shown in Fig. 12. In addition, the display controller 84 displays a bar 98 indicating the elapsed time TP in an annular portion formed by the edges of the small circle 93 and the large circle 94. In a case where the small circle 93 is operated in this display state, the instruction receiving unit 85 receives a test stop instruction. In a case where the test stop instruction is received by the instruction receiving unit 85, the main controller 80 stops the test.

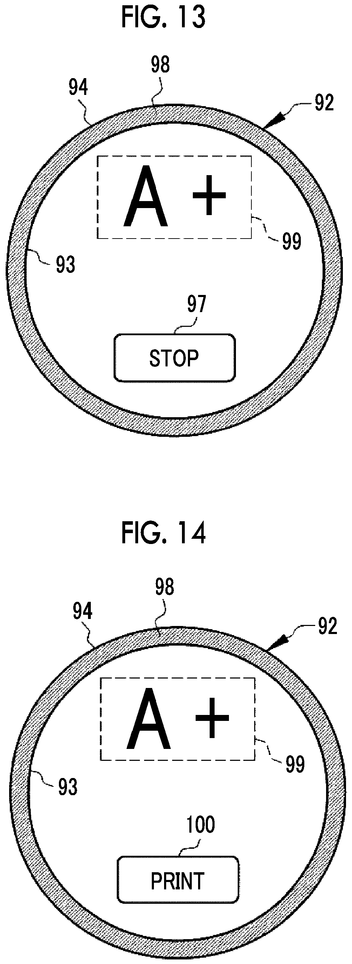

In a case where the first determination result is obtained from the determination unit 82 during a period from the start of the test to the set time TS, the display controller 84 displays an "A+ (A type influenza positive)" mark 99 indicating the first determination result in an upper portion of the small circle 93 as shown in Fig. 13. In a lower portion of the small circle 93, the "STOP" mark 97 is displayed so that the small circle 93 functions as a stop button as in the case of Fig. 12. The bar 98 is displayed in the entire annular portion.

In a case where the small circle 93 is operated in the display state shown in Fig. 13, the instruction receiving unit 85 receives a test stop instruction and the main controller 80 stops the test as in the case of Fig. 12. Then, as shown in Fig. 14, the display controller 84 displays a "print" mark 100 in the lower portion of the small circle 93, instead of the "STOP" mark 97, so that the small circle 93 functions as a print button for giving an instruction to print out a determination result. In a case where the small circle 93 is operated in this display state, the instruction receiving unit 85 receives an instruction to print out a determination result. In a case where an instruction to print out the determination result is received by the instruction receiving unit 85, the printer prints out the determination result on a predetermined sheet.

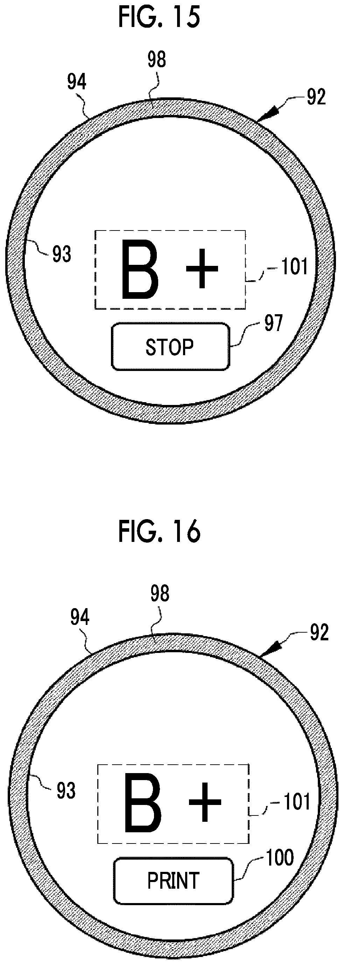

Also in a case where the second determination result is obtained from the determination unit 82 during a period from the start of the test to the set time TS, the process is the same as in Figs. 13 and 14. That is, as shown in Fig. 15, a "B+ (B type influenza positive)" mark 101 indicating the second determination result, instead of the mark 99, is displayed at the center of the small circle 93 so that the small circle 93 functions as a stop button. In a case where the small circle 93 is operated in the display state shown in Fig. 15, the main controller 80 stops the test, and the display controller 84 causes the small circle 93 to function as a print button as shown in Fig. 16.

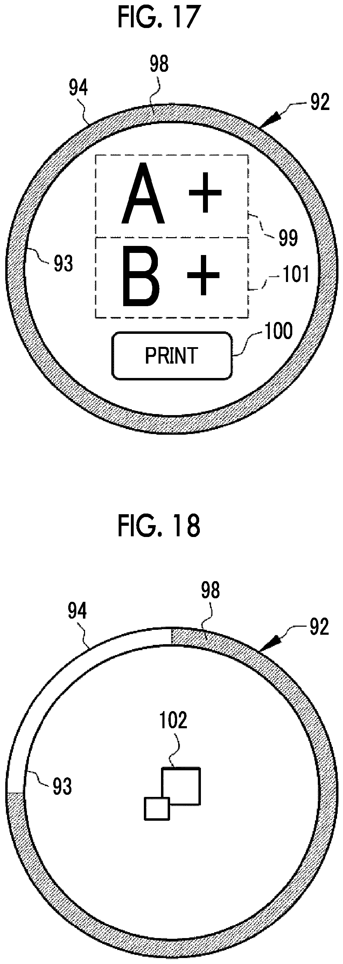

In a case where both the first determination result and the second determination result are obtained from the determination unit 82 during a period from the start of the test to the set time TS, the display controller 84 displays both the mark 99 and the mark 101 in the small circle 93 as shown in Fig. 17. As in the cases of Figs. 14 and 16, the small circle 93 is made to function as a print button.

In a case where the small circle 93 is not operated in the display state in which the small circle 93 functions as a stop button as shown in Figs. 12, 13, and 15, the main controller 80 continues the test.

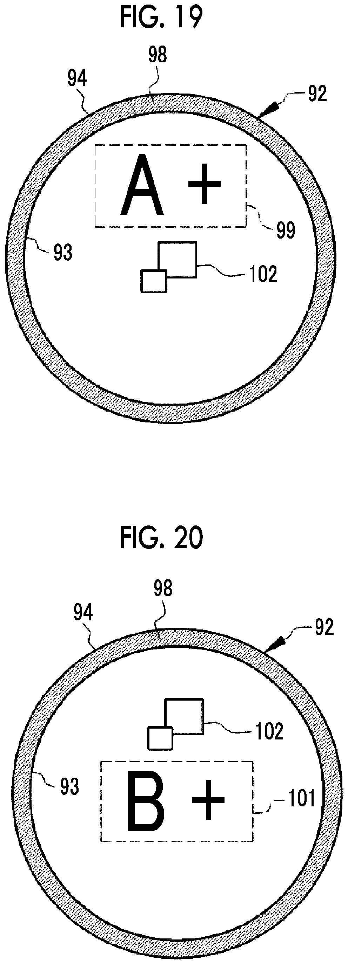

In a case where the small circle 93 is not operated in the display state shown in Fig. 12 and the test is continued and the elapsed time TP exceeds the set time TS and the sensitization processing is started, as shown in Fig. 18, the display controller 84 deletes the "STOP" mark 97 (does not display the stop button) and displays a sensitization mark 102 instead. In a case where the small circle 93 is not operated in the display state shown in Fig. 13 and the test is continued and the elapsed time TP exceeds the set time TS and the sensitization processing is started, as shown in Fig. 19, the display controller 84 displays the sensitization mark 102 instead of the "STOP" mark 97 as in the case of Fig. 18. Similarly, also in a case where the small circle 93 is not operated in the display state shown in Fig. 15 and the sensitization processing is started, as shown in Fig. 20, the display controller 84 displays the sensitization mark 102 instead of the "STOP" mark 97. That is, in a case where the sensitization processing is started, the function of the stop button of the small circle 93 is deactivated by the display controller 84. That is, the display controller 84 corresponds to a function switching unit that switches between activation and deactivation of the function of the stop button.

In the display state shown in Fig. 18, in a case where the state of coloration of the reagents RA and RB is sensitized by the sensitization processing and accordingly the first determination result is obtained from the determination unit 82, the display controller 84 switches the display state of the small circle 93 to the display state shown in Fig. 14. In the display state shown in Fig. 18, in a case where the second determination result is obtained from the determination unit 82, the display controller 84 switches the display state of the small circle 93 to the display state shown in Fig. 16.

In the display state shown in Fig. 18, in a case where both the first determination result and the second determination result are obtained from the determination unit 82, the display controller 84 switches the display state of the small circle 93 to the display state shown in Fig. 17. Also in a case where the second determination result is obtained from the determination unit 82 in the display state shown in Fig. 19 and a case where the first determination result is obtained from the determination unit 82 in the display state shown in Fig. 20, the display controller 84 switches the display state of the small circle 93 to the display state shown in Fig. 17. That is, after the sensitization processing is started, the "STOP" mark 97 is not displayed in the small circle 93. Therefore, after the sensitization processing is started, the small circle 93 does not function as a stop button.

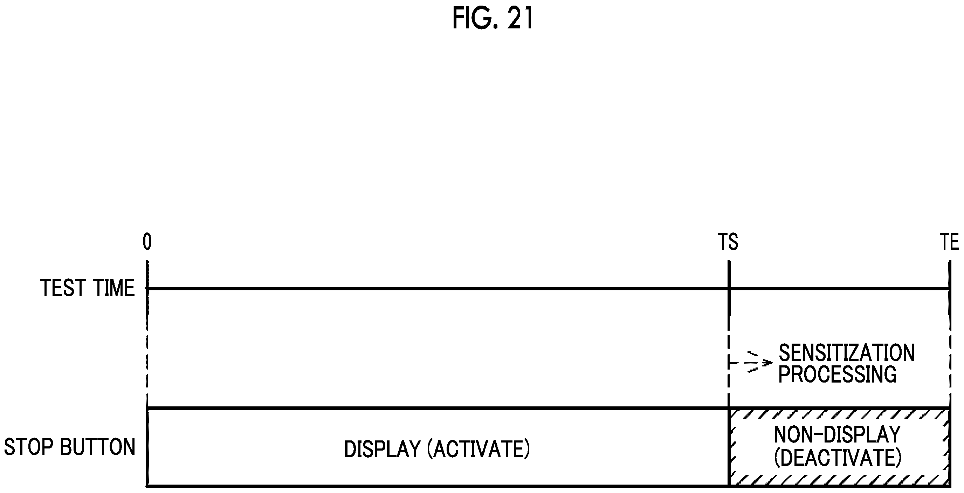

Up to now, various display modes and button functions of the circular display region 92 have been described with reference to Figs. 11 to 20. However, focusing only on the function of the stop button of the small circle 93, the display controller 84 eventually switches between display (function activation) and non-display (function deactivation) of the stop button at the timing shown in Fig. 21. Specifically, the stop button is displayed (function of the stop button is activated) during a period from the start of the test to the set time TS (0 < TP ≤ TS), that is, before the sensitization processing is started, and the stop button is not displayed (function of the stop button is deactivated) during a period from the set time TS to the time TE (TS < TP ≤ TE), that is, after the sensitization processing is started.

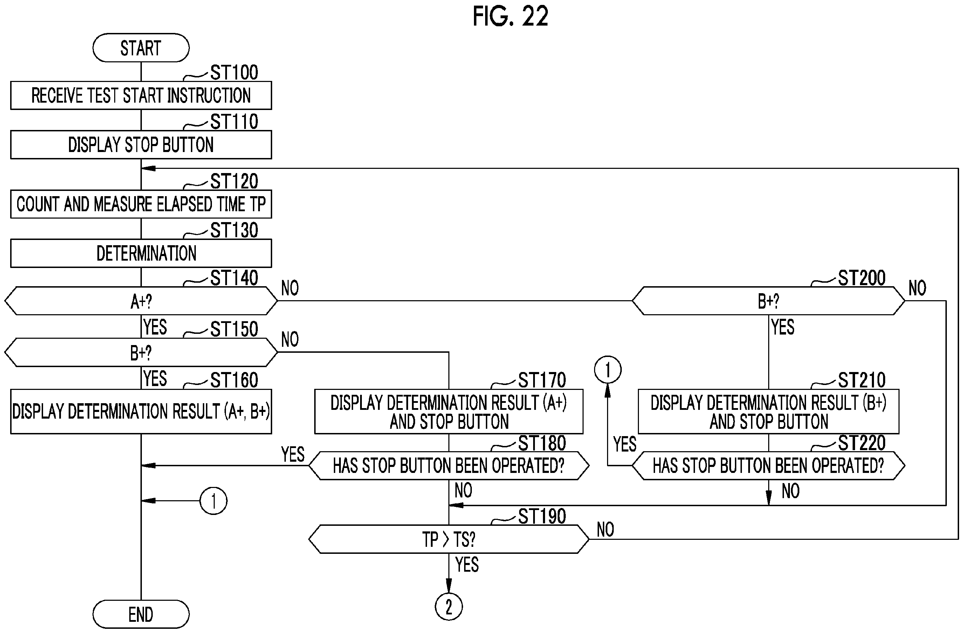

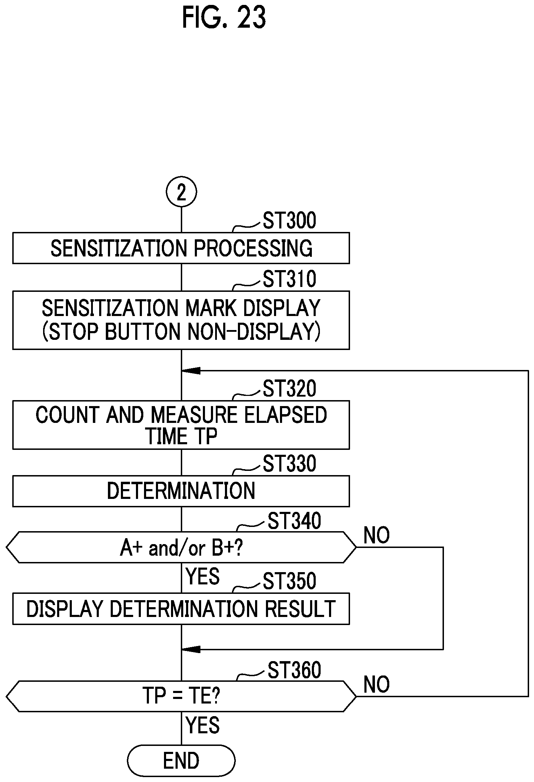

Hereinafter, the operation based on the above configuration will be described with reference to the flowchart shown in Figs. 22 and 23. First, the user applies the sample SSP, which is collected from a patient to be subjected to an immunological test, onto the dropping port 20 of the cartridge 15, opens the lid 17 to load the cartridge 15 into the cartridge loading unit 18, and closes the lid 17. Then, the small circle 93 functioning as a start button in which the "START" mark 96 is displayed, as shown in Fig. 11, is operated. Then, a test start instruction is received by the instruction receiving unit 85 (step ST100). As shown in Fig. 12, in the small circle 93, the "STOP" mark 97 is displayed by the display controller 84.

That is, the small circle 93 functions as a stop button, and the function of the stop button is activated (step ST110).

In a case where the test start instruction is received by the instruction receiving unit 85, counting of the elapsed time TP is started by the main controller 80 and measurement is started by the measurement unit 46 (step ST120). Then, an imaging signal is output from the imaging element 51 of the measurement unit 46 to the determination unit 82. Then, the determination unit 82 derives the coloration state (density, chromaticity) of the reagents RA and RB based on the imaging signal, and determines whether or not the test substances DA and DB are present in the sample SSP from the coloration state (density, chromaticity) of the reagents RA and RB (step ST130).

In a case where both the first determination result indicating that the test substance DA is present in the sample SSP and the second determination result indicating that the test substance DB is present in the sample SSP are obtained in the determination of step ST130 (YES in both steps ST140 and ST150), both the "A+" mark 99 indicating the first determination result and the "B+" mark 101 indicating the second determination result are displayed in the small circle 93 as shown in Fig. 17 (step ST160). In this case, the main controller 80 ends the test without waiting for the elapsed time TP to become the time TE. The user can take out the cartridge 15 from the immunological test apparatus 10.

In a case where only the first determination result is obtained in the determination of step ST130 (YES in step ST140 and NO in step ST150), the "A+" mark 99 is displayed in the small circle 93 as shown in Fig. 13. In addition, the "STOP" mark 97 is displayed in the small circle 93, and subsequently the small circle 93 functions as a stop button (step ST170). In a case where the small circle 93 is operated in this display state (YES in step ST180), a test stop instruction is received by the instruction receiving unit 85, and the test is stopped by the main controller 80.

On the other hand, in a case where the small circle 93 is not operated in the display state shown in Fig. 13 (NO in step ST180) and the elapsed time TP does not exceed the set time TS (0 < TP ≤ TS, NO in step ST190), the process returns to step ST120 to continue the test.

In a case where only the second determination result is obtained in the determination of step ST130 (NO in step ST140 and YES in step ST200), the "B+" mark 101 is displayed in the small circle 93 as shown in Fig. 15. In addition, the "STOP" mark 97 is displayed in the small circle 93, and subsequently the small circle 93 functions as a stop button (step ST210). In a case where the small circle 93 is operated in this display state (YES in step ST220), as in the case of YES in step ST180, a test stop instruction is received by the instruction receiving unit 85, and the test is stopped by the main controller 80.

On the other hand, in a case where the small circle 93 is not operated in the display state shown in Fig.

15 (NO in step ST220) and the elapsed time TP does not exceed the set time TS (0 < TP ≤ TS, NO in step ST190), the process returns to step ST120 to continue the test.

Also in a case where neither the first determination result nor the second determination result is obtained in the determination of step ST130 (NO in both steps ST140 and ST200) and the elapsed time TP does not exceed the set time TS (0 < TP ≤ TS, NO in step ST190), the process returns to step ST120 to continue the test.

In a case where only the first determination result is obtained (YES in step ST140 and NO in step ST150), a case where only the second determination result is obtained (NO in step ST140 and YES in step ST200), and a case where neither the first determination result nor the second determination result is obtained (NO in both steps ST140 and ST200), in a case where the elapsed time TP exceeds the set time TS (TP > TS, YES in step ST190), sensitization processing is performed as shown in step ST300 in Fig. 23 (sensitization processing step).

Specifically, a driving command is output from the main controller 80 to the driving controller 83, and the chemical solution spreading unit 47 (motor 55) is driven by the driving controller 83 that has received the driving command. By the driving of the chemical solution spreading unit 47, the reducing solution pot 37 is crushed by the first pressing piece 61 of the first pressing unit 56, and the reducing solution SR is spread to the test region 31. Then, the sensitizing solution pot 38 is crushed by the second pressing piece 64 of the second pressing unit 57, and the sensitizing solution SSE is spread to the test region 31. This increases the density of coloration of the reagents RA and RB.

In a case where the sensitization processing is started, as shown in Figs. 18 to 20, the "STOP" mark 97 is deleted by the display controller 84 (stop button is not displayed), and instead, the sensitization mark 102 is displayed in the small circle 93 (step ST310, function deactivation step).

Thereafter, as in steps ST120 to ST170 and step ST210 in Fig. 22, counting and measurement of the elapsed time TP (step ST320), determination (step ST330), and display of the obtained determination result (YES in step ST340 and step ST350) are performed. The series of steps ST320 to ST350 are repeated until the elapsed time TP becomes the time TE (TP = TE) and a determination result indicating that the measurement has correctly ended is output from the determination unit 82 (YES in step ST360).

As described above, in a case where the sensitization processing is started, the "STOP" mark 97 of the small circle 93 is deleted to deactivate the function of the stop button of the small circle 93. Accordingly, it is possible to prevent a situation in which the small circle 93 functioning as a stop button is operated and the cartridge 15 is taken out from the immunological test apparatus 10 in a state in which the reducing solution SR or the sensitizing solution SSE spread onto the carrier 30 in the sensitization processing remains as droplets on the carrier 30. Therefore, it is possible to prevent a chemical solution for sensitization processing, such as the reducing solution SR or the sensitizing solution SSE, from adhering to the user.

In a case where the sensitization processing is started, by displaying the "STOP" mark 97 as it is without deleting the "STOP" mark 97 so that no test stop instruction is received by the instruction receiving unit 85 even though the small circle 93 is operated, the test is not stopped after the start of the sensitization processing. In this case, however, since the "STOP" mark 97 is displayed, the user may misunderstand that the test can be stopped. Accordingly, in a case where the test is not stopped even though the small circle 93 is operated, the user may misunderstand the situation as an apparatus failure or may misunderstand the situation as the lack of operation power and operates the small circle 93 strongly. In the worst case, the user may open the lid 17 and forcibly takes out the cartridge 15. That is, various problems may occur. This problem is not limited to the case where the stop button is displayed on the touch panel 19. For example, even in a case where a push type mechanical stop button is provided in the apparatus main body 10A, the problem can similarly occur since the stop button itself remains even in a case where the function of the stop button is deactivated.

On the other hand, in this example, the function of the stop button is deactivated by deleting the "STOP" mark 97 (displaying no stop button). Therefore, since the user does not misunderstand that the test can be stopped, the above-described problems do not occur.

As in this example, in a case where a solution of ammonium iron sulfate that is a divalent iron ion-containing compound is used as the reducing solution SR and a solution of silver nitrate that is a silver ion-containing compound is used as the sensitizing solution SSE, there is a concern of chemical burns or staining due to adhesion to the user's hand or the like, but such a concern can be completely eliminated.

The sensitization processing is started in a case where at least one of the first determination result indicating that the test substance DA is present in the sample SSP or the second determination result indicating that the test substance DB is present in the sample SSP is not obtained during a period from the start of the test to the set time TS. Accordingly, in a case where there is only a very small amount of test substances DA and DB in the sample SSP and the density of coloration of the reagents RA and RB is very low, that is, only in a case where it is certainly necessary to increase the density of coloration of the reagents RA and RB, the sensitization processing can be performed.

In medical institutions, a large number of qualitative reaction tests of influenza are performed in winter in which influenza spreads. For this reason, the user has a relatively high chance of touching the cartridge 15.

Therefore, it is thought that a probability that a chemical solution for sensitization processing will adhere to the user is also relatively high. In this example, the reagents RA and RB contain an influenza virus antibody, and the immunological test apparatus 10 performs a qualitative reaction test of influenza. Therefore, the effect that the chemical solution for sensitization processing can be prevented from adhering to the user is particularly effective.

After the passage of a solution absorption time until the reducing solution SR and the sensitizing solution SSE are completely absorbed by the carrier 30 and remaining droplets disappear from the spreading of the reducing solution SR and the sensitizing solution SSE onto the carrier 30, the stop button may be displayed (function of the stop button may be activated). Unless there are remaining droplets on the carrier 30, there is no possibility that the reducing solution SR or the sensitizing solution SSE will adhere to the user even though the test is stopped and the cartridge 15 is taken out. Therefore, there is no problem even in a case where the stop button is displayed (function of the stop button is activated).

In addition to not displaying the stop button (deactivating the function of the stop button), the lid 17 may be locked so that the cartridge 15 is not physically taken out. It is necessary to provide a lock mechanism on the lid 17. In this case, it is possible to more reliably prevent a chemical solution for sensitization processing from adhering to the user.

[Aspect according to the second disclosure.]

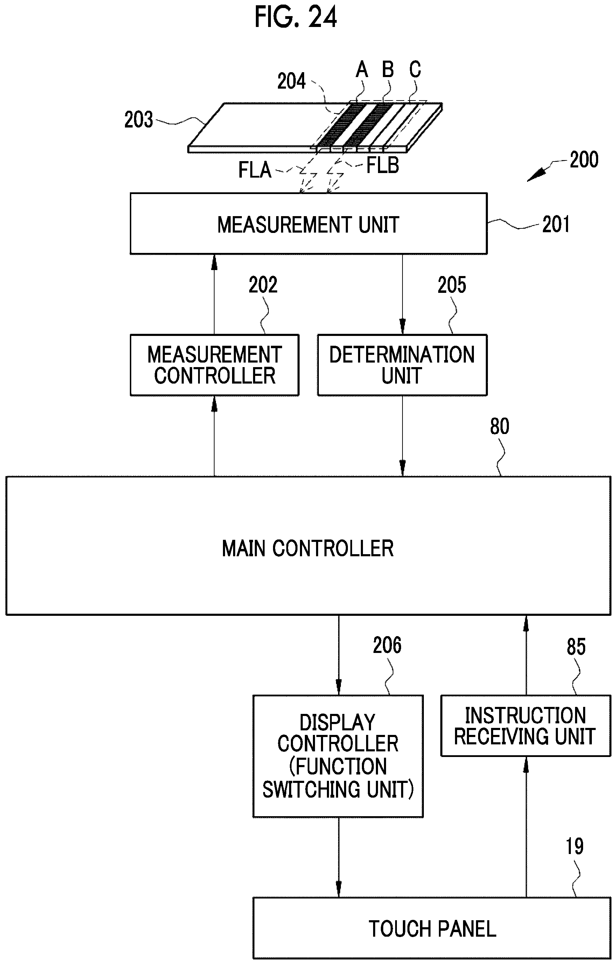

In an an aspect according to the second disclosure, shown in Figs. 24 to 27, the function of the stop button is deactivated until a determination result indicating that a test substance is present in the sample SSP is obtained from the start of the test, and the function of the stop button is activated in a case where the determination result indicating that a test substance is present in the sample SSP is obtained. Hereinafter, the same reference numerals are given to the same components as those of the embodiment according to the first invention described above, and the description thereof will be omitted.

In Fig. 24, an immunological test apparatus 200 of the aspect according to the second disclosure is different from the configuration of the immunological test apparatus 10 of the embodiment according to the first invention in that the chemical solution spreading unit 47 and the driving controller 83 are not provided. A measurement unit 201 measures fluorescence FLA and FLB emitted from reagents RA and RB of test lines A and B of a test region 204 of a carrier 203 under the control of a measurement controller 202. A determination unit 205 determines whether or not the test substances DA and DB are present in the sample SSP from the state of emission of the fluorescence FLA and FLB.

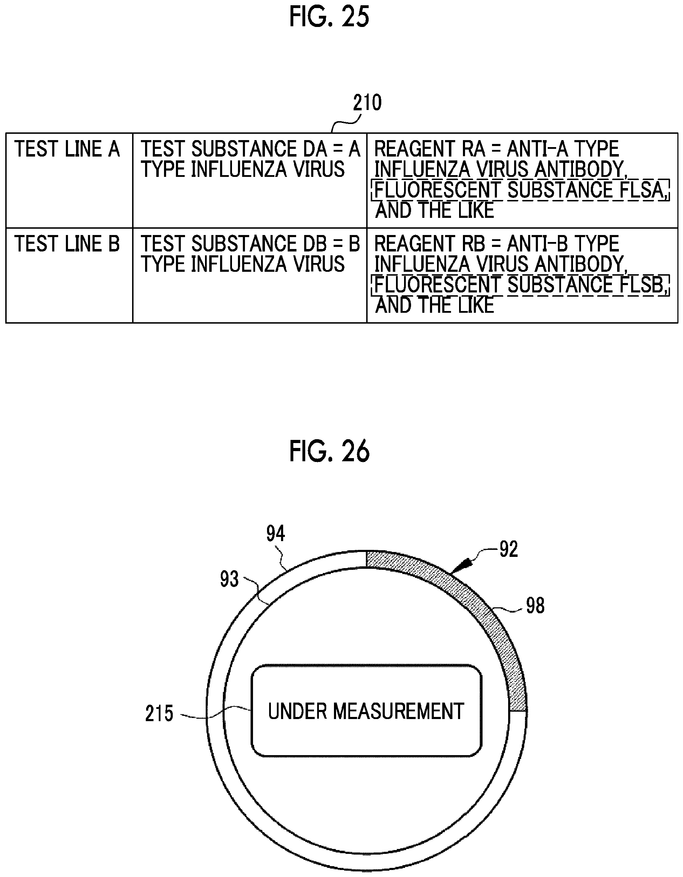

As shown in a table 210 of Fig. 25, in the aspect according to the second disclosure, as in the embodiment according to the first invention, the test substance DA is A type influenza virus, and the test substance DB is B type influenza virus. The reagent RA is configured to further contain a fluorescent substance FLSA in addition to the anti-A type influenza virus antibody and the like in the embodiment according to the first invention. The reagent RB is configured to further contain a fluorescent substance FLSB in addition to the anti-B type influenza virus antibody and the like in the embodiment according to the first invention. The fluorescent substance FLSA emits the fluorescence FLA, and the fluorescent substance FLSB emits the fluorescence FLB.

In a case where a cartridge (not shown) containing the carrier 203 (having the sample SSP dropped thereon) is loaded, a display controller 206 displays the "START" mark 96 shown in Fig. 11 in the small circle 93 so that the small circle 93 functions as a start button, as in the embodiment according to the first invention.

In the embodiment according to the first invention, after the small circle 93 is operated in the display state shown in Fig. 11 and the test start instruction is received by the instruction receiving unit 85 and the test is started, the "STOP" mark 97 is displayed in the small circle 93 so that the small circle 93 functions as a stop button as shown in Fig. 12. On the other hand, in the aspect according to the second disclosure, after the test is started, the display controller 206 displays an "under measurement" mark 215 at the center of the small circle 93 as shown in Fig. 26. That is, the small circle 93 does not function as a stop button.

Thereafter, in a case where the first determination result indicating that the test substance DA is present in the sample SSP or the second determination result indicating that the test substance DB is present in the sample SSP is obtained from the determination unit 205, the display controller 206 displays the "A+" mark 99 indicating the first determination result or the "B+" mark 101 indicating the second determination result and the "STOP" mark 97 in the small circle 93 as in Figs. 13 and 15. That is, in a case where a determination result indicating that at least one of the test substances DA and DB is present in the sample SSP is obtained, the stop button is displayed (function of the stop button is activated).

In a case where both the first determination result and the second determination result are obtained from the determination unit 205, the display controller 206 displays both the mark 99 and the mark 101 in the small circle 93 as in Fig. 17.

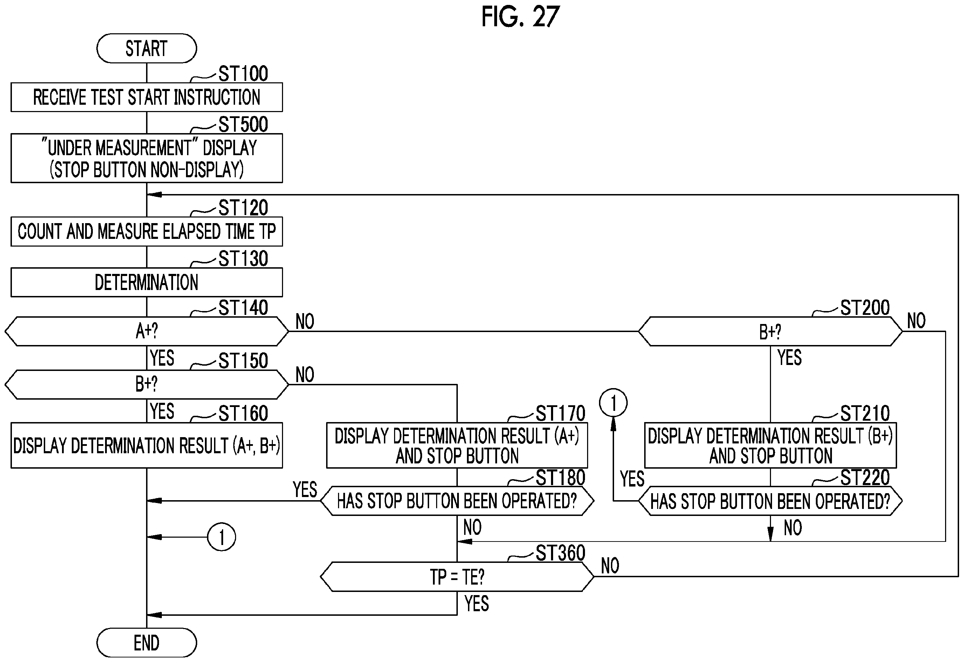

The operation of the aspect according to the second disclosure will be described with reference to the flowchart shown in Fig. 27. First, after a test start instruction is received by the instruction receiving unit 85 (step ST100), the display controller 206 displays the "under measurement" mark 215 in the small circle 93 as shown in Fig. 26. That is, since a stop button is not displayed in the small circle 93, the small circle 93 does not function as a stop button (step S500, function deactivation step).

In a case where only the first determination result is obtained in the determination of step ST130 (YES in step ST140 and NO in step ST150), the "A+" mark 99 and the "STOP" mark 97 are displayed in the small circle 93 as shown in Fig. 13. That is, the small circle 93 functions as a stop button, and the function of the stop button is activated (step ST170, function activation step). In a case where the small circle 93 is operated in this display state (YES in step ST180), a test stop instruction is received by the instruction receiving unit 85, and the test is stopped by the main controller 80.

On the other hand, in a case where the small circle 93 is not operated in the display state shown in Fig. 13 (NO in step ST180) and the elapsed time TP is not the time TE (TP ≠ TE, NO in step ST360), the process returns to step ST120 to continue the test.

In a case where only the second determination result is obtained in the determination of step ST130 (NO in step ST140 and YES in step ST200), as shown in Fig. 15, the "B+" mark 101 and the "STOP" mark 97 are displayed in the small circle 93, the small circle 93 functions as a stop button, and the function of the stop button is activated (step ST210, function activation step). In a case where the small circle 93 is operated in this display state (YES in step ST220), as in the case of YES in step ST180, a test stop instruction is received by the instruction receiving unit 85, and the test is stopped by the main controller 80.

On the other hand, in a case where the small circle 93 is not operated in the display state shown in Fig. 15 (NO in step ST220) and the elapsed time TP is not the time TE (TP ≠ TE, NO in step ST360), the process returns to step ST120 to continue the test.

As described above, the stop button is not displayed (function of the stop button is deactivated) until a determination result indicating that at least one of the test substances DA and DB is present in the sample SSP is obtained from the start of the test, and the stop button is displayed (function of the stop button is activated) in a case where the determination result indicating that at least one of the test substances DA and DB is present in the sample SSP is obtained. Therefore, in a case where a determination result desired by the user between the first determination result and the second determination result is obtained, the test can be stopped promptly.

In the qualitative reaction test of influenza, it does not matter so much whether the patient is infected with either A type influenza virus or B type influenza virus. This is because, regardless of A type influenza virus or B type influenza virus, treatment methods such as the type, dose, and usage of the drug to be administered are generally common. For this reason, in a case where the infection with either A type influenza virus or B type influenza virus is found, the minimum purpose of the qualitative reaction test of influenza can be achieved.

Therefore, in a case where a determination result indicating that at least one of the test substances DA and DB is present in the sample SSP is obtained, operating the stop button to stop the test does not cause any problem. Therefore, also in the embodiment according to the first invention, in a case where a determination result indicating that at least one of the test substances DA and DB is present in the sample SSP is obtained, it is possible to display the stop button (activate the function of the stop button) so that the test is stopped, as shown in Figs. 13 and 15.

In each of the embodiment described above, the hardware structures of processing units for executing various kinds of processing, such as the driving controller 83 and the display controllers 84 and 206 as function switching units, are various processors shown below. The various processors include a central processing unit (CPU) that is a general-purpose processor that executes software (program) to function as various processing units, a programmable logic device (PLD) that is a processor whose circuit configuration can be changed after manufacture, such as a field programmable gate array (FPGA), and a dedicated electric circuit that is a processor having a circuit configuration that is designed for exclusive use in order to execute specific processing, such as an application specific integrated circuit (ASIC).

One processing unit may be configured by one of various processors, or may be a combination of two or more processors of the same type or different types (for example, a combination of a plurality of FPGAs or a combination of a CPU and an FPGA). Alternatively, a plurality of processing units may be configured by one processor. As an example of configuring a plurality of processing units using one processor, first, there is a form in which one processor is configured by a combination of one or more CPUs and software and this processor functions as a plurality of processing units. Second, as represented by a system on chip (SoC) or the like, there is a form of using a processor for realizing the function of the entire system including a plurality of processing units with one integrated circuit (IC) chip. Thus, various processing units are configured by using one or more of the above-described various processors as a hardware structure.

In addition, the hardware structure of these various processors is an electrical circuit (circuitry) in the form of a combination of circuit elements, such as semiconductor elements.

In the present invention, it is also possible to appropriately combine the above-described various embodiments or various modification examples. Without being limited to the embodiments described above, it is needless to say that various configurations can be adopted without departing from the scope of the present invention as defined in the appended claims. For example, an example has been described in which measurement and determination are repeated until the elapsed time TP reaches the time TE after the sensitization processing. However, measurement and determination after the sensitization processing may be performed only once. The test substance is not limited to influenza virus. The test substance may be adenovirus, rotavirus, hepatitis virus, pneumococci, and the like. The immunological test is not limited to the qualitative reaction test.

Explanation of References

    10, 200: immunological test apparatus
    10A: apparatus main body
    15: cartridge
    15A: case
    16: opening
    17: lid
    18: cartridge loading unit
    19: touch panel
    20: dropping port
    21: label
    30, 203: carrier
    31, 204: test region
    32, 33: observation window
    35: solution feeding pad
    36: solution absorbing pad
    37: reducing solution pot
    38: sensitizing solution pot
    45: guide rail
    46, 201: measurement unit
    47: chemical solution spreading unit
    50: light source
    51: imaging element
    55: motor
    56: first pressing unit
    57: second pressing unit
    60, 63: first and second arms
    60A: shaft
    61, 64: first and second pressing piece
    62, 65: first and second cams
    70, 75, 210: table
    80: main controller
    81, 202: measurement controller
    82, 205: determination unit
    83: driving controller
    84, 206: display controller (function switching unit)
    85: instruction receiving unit
    90: display screen
    91: character display region
    92: circular display region
    93: small circle
    94: large circle
    96: "START" mark
    97: "STOP" mark
    98: bar
    99: "A+" mark
    100: "PRINT" mark
    101: "B+" mark
    102: sensitization mark
    215: "under measurement" mark
    A, B: test line

    C: control line
    SSP: sample
    SR: reducing solution
    SSE: sensitizing solution
    DA, DB: test substance
    RA, RB: reagent
    TP: elapsed time
    TS: set time
    TE: time taken for sample to reach control line
    ST100 to S220, ST300 to ST360, ST500: step

**Claims**

1. An immunological test apparatus (10) that receives a carrier (30) holding a reagent (RA, RB), which is combined with a test substance (DA, DB) to be colored, and having a sample (SSP) dropped thereon, measures a state of the coloration, and determines whether or not the test substance (DA, DB) is present in the sample based on a result of the measurement, the apparatus (10) comprising:

    a chemical solution spreading unit (47) that performs sensitization processing for spreading a chemical solution for sensitizing a state of the coloration onto the carrier by increasing the density of coloration, wherein the chemical solution includes a reducing solution (SR) and a sensitizing solution (SSE);
    a driving controller (83) that controls driving of the chemical solution spreading unit (47);
    a stop button that gives an instruction to stop a test; and
    a function switching unit (84) that performs switching between activation and deactivation of a function of the stop button and that deactivates the function of the stop button in a case where the driving controller (83) drives the chemical solution spreading unit to start the sensitization processing.

2. The immunological test apparatus (10) according to claim 1,

    wherein the function switching unit (84) is a display controller that performs control to display the stop button on a touch panel (19), and
    the stop button is not displayed in a case where the sensitization processing is started.

3. The immunological test apparatus (10) according to claim 1 or 2,
    wherein the driving controller(83) drives the chemical solution spreading unit (47) to start the sensitization processing in a case where a determination result indicating that the test substance is present in the sample is not obtained during a period from test start to a set time set in advance.

4. The immunological test apparatus (10) according to any one of claims 1 to 3, wherein there are two types of the chemical solutions that are a divalent iron ion-containing compound and a solution of a silver ion-containing compound.

5. The immunological test apparatus (10) according to any one of claims 1 to 4, wherein the reagent contains an antibody of influenza virus.

## Patentansprüche

1. Immunologische Testvorrichtung (10), die einen Träger (30) aufnimmt, welcher ein mit einer zu färbenden Testsubstanz (DA, DB) kombiniertes Reagens hält, worauf eine Probe (SSP) getropft wird, die einen Zustand der Färbung misst, und die aufgrund eines Messergebnisses feststellt, ob die Testsubstanz (DA, DB) in der Probe vorhanden ist, umfassend:

eine Verteilungseinheit (47) für eine chemische Lösung, die eine Sensibilisierungsverarbeitung ausführt zum Verteilen einer chemischen Lösung zum Sensibilisieren eines Färbungszustands auf dem Träger, indem die Färbungsdichte erhöht wird, wobei die chemische Lösung eine reduzierende Lösung (SR) und eine sensibilisierende Lösung (SSE) enthält;
eine Treibersteuerung (83), die ein Treiben der Verteilungseinheit (47) für eine chemische Lösung steuert;
eine Stopptaste, die einen Befehl zum Stoppen eines Tests liefert; und
eine Funktionsumschalteinheit (84), die eine Umschaltung bewirkt zwischen einer Aktivierung und einer Deaktivierung einer Funktion der Stopptaste, und die die Funktion der Stopptaste dann deaktiviert, wenn die Treibersteuerung (83) die Verteilungseinheit für chemische Lösung treibt, um die Sensibilisierungsverarbeitung zu starten.

2. Immunologische Testvorrichtung (10) nach Anspruch 1,

bei der die Funktionsumschalteinheit (84) eine Anzeigesteuerung ist, die eine Steuerung zum Anzeigen der Stopptaste auf einem Touchpanel (19) ausführt, und
die Stopptaste dann nicht angezeigt wird, wenn die Sensibilisierungsverarbeitung gestartet wird.

3. Immunologische Testvorrichtung (10) nach Anspruch 1 oder 2,
bei der die Treibersteuerung (83) die Verteilungseinheit (47) für chemische Lösung treibt, um die Sensibilisierungsverarbeitung zu starten, falls ein Feststellungsergebnis, das angibt, dass die Testsubstanz in der Probe vorhanden ist, nicht innerhalb einer Zeitspanne zwischen dem Test-Start und einer vorab eingestellten Zeit erhalten wird.

4. Immunologische Testvorrichtung (10) nach einem der Ansprüche 1 bis 3,
bei der es zwei Arten chemischer Lösungen gibt, bei denen es sich um eine zweiwertige Eisenionen enthaltende Verbindung und eine Lösung einer silberionenhaltigen Verbindung handelt.

5. Immunologische Testvorrichtung (10) nach einem der Ansprüche 1 bis 4,
bei der das Reagens einen Antikörper eines Influenzavirus' enthält.

## Revendications

1. Appareil d'essai immunologique (10), lequel reçoit un support (30) contenant un réactif (RA, RB), lequel est combiné avec une substance d'essai (DA, DB) à colorer, et présentant un échantillon (SSP) déposée sur celui-ci, mesure un état de la coloration, et détermine si oui ou non la substance d'essai (DA, DB) est présente dans l'échantillon sur la base d'un résultat de la mesure, l'appareil (10) comprenant :

une unité d'étalement de solution chimique (47), laquelle réalise un traitement de sensibilisation pour étaler une solution chimique pour sensibiliser un état de la coloration sur le support en augmentant la densité de coloration, dans lequel la solution chimique inclut une solution de réduction (SR) et une solution de sensibilisation (SSE) ;
un contrôleur d'entraînement (83), lequel commande l'entraînement de l'unité d'étalement de solution chimique (47) ;
un bouton d'arrêt, lequel donne une instruction pour arrêter un essai, et
une unité de commutation de fonctionnement (84), laquelle réalise une commutation entre l'activation et la désactivation du fonctionnement du bouton d'arrêt, et laquelle désactive le fonctionnement du bouton d'arrêt dans un cas où le contrôleur d'entraînement (83) entraîne l'unité d'étalement de solution chimique afin de lancer le traitement de sensibilisation.

2. Appareil d'essai immunologique (10) selon la revendication 1,
dans lequel l'unité de commutation de fonctionnement (84) est un contrôleur d'affichage, lequel réalise une commande pour afficher le bouton d'arrêt sur un écran tactile (19), et

le bouton d'arrêt n'est pas affiché dans un cas où le traitement de sensibilisation est lancé.

3. Appareil d'essai immunologique (10) selon la revendication 1 ou 2,

dans lequel le contrôleur d'entraînement (83) entraîne l'unité d'étalement de solution chimique (47) afin de lancer le traitement de sensibilisation dans un cas où un résultat de détermination indiquant que la substance d'essai est présente dans l'échantillon n'est pas obtenu durant une période allant du lancement d'essai à un temps réglé, réglé à l'avance.

4. Appareil d'essai immunologique (10) selon l'une quelconque des revendications 1 à 3,

dans lequel il existe deux types pour les solutions chimiques, lesquels sont un composé contenant des ions fer divalents et une solution de composé contenant des ions argent.

5. Appareil d'essai immunologique (10) selon l'une quelconque des revendications 1 à 4, dans lequel le réactif contient un anticorps du virus de la grippe.

FIG. 1

EP 3 584 578 B1

## FIG. 2

EP 3 584 578 B1

FIG. 3

## FIG. 4

## FIG. 5

## FIG. 6

## FIG. 7

| TEST LINE A | TEST SUBSTANCE DA = A TYPE INFLUENZA VIRUS | REAGENT RA = ANTI-A TYPE INFLUENZA VIRUS ANTIBODY AND THE LIKE |
|---|---|---|
| TEST LINE B | TEST SUBSTANCE DB = B TYPE INFLUENZA VIRUS | REAGENT RB = ANTI-B TYPE INFLUENZA VIRUS ANTIBODY AND THE LIKE |

## FIG. 8

| REDUCING SOLUTION SR | AMMONIUM IRON SULFATE SOLUTION |
|---|---|
| SENSITIZING SOLUTION SSE | SILVER NITRATE SOLUTION |

## FIG. 9

# FIG. 10

Flu

23456789

00:00 /15:00

EP 3 584 578 B1

## FIG. 11

## FIG. 12

## FIG. 13

A +

STOP

## FIG. 14

A +

PRINT

## FIG. 15

98
94
92
93
B +
101
STOP
97

## FIG. 16

98
94
92
93
B +
101
PRINT
100

## FIG. 17

A +

B +

PRINT

## FIG. 18

## FIG. 19

## FIG. 20

# FIG. 21

EP 3 584 578 B1

**FIG. 22**

START

ST100 — RECEIVE TEST START INSTRUCTION

ST110 — DISPLAY STOP BUTTON

ST120 — COUNT AND MEASURE ELAPSED TIME TP

ST130 — DETERMINATION

ST140 — A+? → NO → ST200 — B+? → NO

YES — ST150 — B+? → NO

YES — ST160 — DISPLAY DETERMINATION RESULT (A+, B+)

ST170 — DISPLAY DETERMINATION RESULT (A+) AND STOP BUTTON

ST200 — B+? → NO

YES — ST210 — DISPLAY DETERMINATION RESULT (B+) AND STOP BUTTON

ST180 — HAS STOP BUTTON BEEN OPERATED? — YES / NO

ST220 — HAS STOP BUTTON BEEN OPERATED? — YES / NO

ST190 — TP > TS? — NO / YES

END

EP 3 584 578 B1

# FIG. 23

```
                    ( 2 )
                              ST300
      ┌─────────────────────────────┐
      │   SENSITIZATION PROCESSING   │
      └─────────────────────────────┘
                              ST310
      ┌─────────────────────────────┐
      │   SENSITIZATION MARK DISPLAY │
      │   (STOP BUTTON NON-DISPLAY)  │
      └─────────────────────────────┘

                              ST320
      ┌─────────────────────────────┐
      │  COUNT AND MEASURE ELAPSED   │
      │           TIME TP            │
      └─────────────────────────────┘
                              ST330
      ┌─────────────────────────────┐
      │        DETERMINATION         │
      └─────────────────────────────┘
                              ST340
      <      A+ and/or B+?       >────NO
              YES       ST350
      ┌─────────────────────────────┐
      │  DISPLAY DETERMINATION RESULT │
      └─────────────────────────────┘

                              ST360
      <        TP = TE?          >────NO
              YES
          (  END  )
```

# FIG. 24

## FIG. 25

210

| TEST LINE A | TEST SUBSTANCE DA = A TYPE INFLUENZA VIRUS | REAGENT RA = ANTI-A TYPE INFLUENZA VIRUS ANTIBODY, FLUORESCENT SUBSTANCE FLSA, AND THE LIKE |
|---|---|---|
| TEST LINE B | TEST SUBSTANCE DB = B TYPE INFLUENZA VIRUS | REAGENT RB = ANTI-B TYPE INFLUENZA VIRUS ANTIBODY, FLUORESCENT SUBSTANCE FLSB, AND THE LIKE |

## FIG. 26

94

93

92

98

215

UNDER MEASUREMENT

# FIG. 27

START

ST100
RECEIVE TEST START INSTRUCTION

ST500
"UNDER MEASUREMENT" DISPLAY
(STOP BUTTON NON-DISPLAY)

ST120
COUNT AND MEASURE ELAPSED TIME TP

ST130
DETERMINATION

ST140
A+? — NO

ST200
B+? — NO

YES

ST150
B+? — NO

YES

ST160
DISPLAY DETERMINATION RESULT (A+, B+)

ST170
DISPLAY DETERMINATION RESULT (A+)
AND STOP BUTTON

ST210
DISPLAY DETERMINATION RESULT (B+)
AND STOP BUTTON

ST180
HAS STOP BUTTON BEEN OPERATED? — YES

NO

ST220
HAS STOP BUTTON BEEN OPERATED? — YES

NO

ST360
TP = TE? — NO

YES

END

EP 3 584 578 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012103150 A

- US 8474303 B2

**Non-patent literature cited in the description**

- **M. MORI et al.** *Development of a highly sensitive immunochromatographic detection kit for seasonal influenza virus using silver amplification*